# EUROPÄISCHE PATENTSCHRIFT

(11) **EP 0 770 309 B1**
(45) Veröffentlichungstag und Bekanntmachung des Hinweises auf die Patenterteilung: **05.01.2000**
(21) Anmeldenummer: 95920845.5
(22) Anmeldetag: 19.05.1995
(51) Int. Cl.: H04N 13/00, G02B 23/24, A61B 1/00

(54) **3D-VIDEO-ENDOSKOP**
3D VIDEO ENDOSCOPE
ENDOSCOPE VIDEO TRIDIMENSIONNEL

(30) Priorität: 08.07.1994 DE 4424114
(43) Veröffentlichungstag der Anmeldung: 02.05.1997
(73) Patentinhaber: Forschungszentrum Karlsruhe GmbH, 76133 Karlsruhe (DE)
(72) Erfinder: BECKER, Heinz, D-76646 Bruchsal (DE)
(74) Vertreter: Rückert, Friedrich, Dr.
(86) Internationale Anmeldenummer: EP9501922
(87) Internationale Veröffentlichungsnummer: WO9602114

(56) Entgegenhaltungen:
- EP-A- 0 222 293
- EP-A- 0 269 075
- WO-A-87/01896
- DE-A- 3 806 190
- US-A- 4 862 873
- US-A- 4 924 853

## Beschreibung

Die Erfindung betrifft ein 3D-Video-Endoskop. Mit ihm kann der Anwender unter gewohnter dreidimensionaler Sicht in nicht unmittelbar zugänglichen Hohlräumen beobachten.

In der DE 38 06 190 wird ein 3-D-Video-System mit kontinuierlicher, zweikanaliger Bildaufnahme und einer Bildwiedergabe über eine Polarisationseinrichtung beschrieben. Das 3D-Video-System hat in den beiden optischen Kanälen jeweils nur eine Strahlumlenkeinrichtung, somit sind optisch nur Spiegelbilder des Gegenstands erhältlich. Die Polarisationseinrichtung erlaubt darüber hinaus nicht, während der Betrachtung des Monitors den Kopf stärker aus der vorgesehenen Position zu schwenken.

Die DE 36 40 731 zeigt dagegen eine opto-elektronische 3D-Aufnahmeeinrichtung mit optischen Ventilen auch unmittelbar vor den Augen des Betrachters. Die optischen Achsen der Objektive bilden den Konvergenzwinkel oder Schielwinkel, der für das räumliche Sehen mit Tiefeneindruck wesentlich ist. Jeder Strahl wird zweimal über Spiegel oder Prismen umgelenkt, bis er auf den optischen Eingang der einen Video-Kamera trifft. Somit werden seitenrichtige Bilder wiedergegeben. In jedem der beiden Strahlengänge befindet sich ein optisches Ventil, so daß abwechselnd das rechte und linke Bild Durchlaß findet. Die Aufnahmekapazität der einen Video-Kamera steht für beide Bilder stets ausschließlich zur Verfügung.

In der Druckschrift der Firma OPTIKON aus dem Jahr 1993 wird ein vollständiges und einsetzbares 3D-Video-System für die endoskopischen Chirurgie beschrieben, das im Prinzip genauso wie die oben erwähnte Literaturstelle mit zwei optischen Kanälen ausgestattet ist. Vor beiden Strahleintrittsflächen befindet sich je eine Vorlinse, die die davorliegende Umgebung abbilden. Beide optischen Kanäle führen auf je eine CCD-Kamera, die am proximalen Ende angeflanscht sind. Die Kamerasteuerungen sind mit einer 3D-Elektronik verbunden. Durch diese werden die Bilder der beiden Kameras mit einer Frequenz von 100 - 120 Hz im zeitlichen Wechsel auf einem Monitor dargestellt.

Der Aufbau des 3D-Video-Endoskops besteht im wesentlichen aus den zwei optischen Kanälen mit Vorsatzlinsen, die in einem Schaft untergebracht sind, und den auf diesen Schaft aufgesetzten beiden Kameras oder Kameraköpfen.

In einer weiteren Literaturstelle, der US 4,924,853, wird ein 3D-Video-Endoskop beschrieben, das zwei Vorlinsen mit je einer optischen Achse aufweist. Im optischen Strahlengang nach den beiden Vorlinsen befinden sich steuerbare optische Schalter, die im gleichen Rhythmus komplementär abwechselnd transparent und opak gesteuert werden. Daran schließt sich eine Strahlumlenkeinrichtung an, die die optischen Achsen der beiden Strahlengänge auf der optische Achse einer optoelektronischen Kamera zusammenführt. Dadurch wird die Kamera an ihrem Ausgang im Rhythmus der beiden optischen Schalter einmal die elektrischen Signale des linken und dann die des rechten Bildes zur weiteren Video-Bearbeitung bereitstellen. Die Strahlumlenkeinrichtung besteht zunächst aus einem v-förmig prismatischen Strahlumlenkungsgruppe zur Strahlaufweitung und dann aus einer weiteren Strahlumlenkungsgruppe zur Zusammenführung beider Strahlachsen, die aus diskreten Prismen aufgebaut ist. Zwischen beiden Strahlumlenkungsgruppen befindet sich der jeweilige optische Schalter.

In der WO 87/01896 wird ebenfalls eine v-förmig prismatische Strahlführungsvorrichtung gezeigt und beschrieben, in der ein eintreffendes Lichtbündel in drei primäre Farbbündel aufgeteilt wird. Hier dient diese Vorrichtung nur zur Aufweitung der farbanteiligen optischen Achsen.

Der Erfindung liegt die Aufgabe zugrunde, ein 3D-Video-Endoskop bereitzustellen, das ohne Beeinträchtigung in der Bildqualität räumlich schlank gebaut werden kann und vor allem an der Stelle der opto-elektronischen Übergangs raumsparend aufgebaut ist, so daß enge Durchführungsbereiche passierbar bleiben.

Die Aufgabenstellung kommt vornehmlich aus dem Bereich der minimal invasiven Chirurgie, darüber hinaus aber ganz allgemein auch aus unterschiedlichen Bereichen der Hohlrauminspektion. Aus dem Bereich der Kanalinspektionssysteme kommt die Forderung, in unzugänglichen Rohrsystemen ein Inspektionssystem mit 3D-Aufnahmetechnik einsetzen zu können, mit dem eine wirklichkeitsgetreue, räumliche Darstellung der Szene möglich ist.

Diese Aufgabe wird erfindungsgemäß durch den Aufbau eines Endoskops gemäß dem Anspruch 1 gelöst. Paarweise treten dort nur noch die Vorlinsen und die beiden ansteuerbaren optischen Schalter im optischen Strahlengang auf.

Der Unteranspruch 2 kennzeichnet eine an sich bekannte Bauformen, die je nach Einsatz vorteilhaft oder zweckmäßig ist, nämlich die des mehr oder weniger abgeknickten Schafts.

Das in Anspruch 1 gekennzeichnete 3D-Video-Endoskop hat nur noch einen Kamerakopf, an dessen Ausgang die elektrischen Signale zur Videoverarbeitung anstehen, d. h. im Schaft werden elektrische Signale über Kabel weitergeleitet. Damit entfällt ein optischer Übertragungskanal. Der Schaft kann steif, flexibel oder geknickt sein.

Hervorzuheben ist das Prinzp der Strahlumlenkung durch zweimalige Reflexion des reflektierten Strahls, wodurch ein seitenkonformes Bild aufrechterhalten wird.

Als optische Schalter haben sich elektro-optische Blenden (Anspruch 3) bewährt, die einfach und zuverlässig vom Kamerakopf aus gesteuert werden können. Dadurch besteht eine sichere Zuordnung zum linken oder rechten Bild (Video-Halbbild).

Das erfindungsgemäße Endoskop zeichnet sich durch Platzeinsparung und erheblich geringeren finanziellen Aufwand durch die Einsparung einer Kamera und die notwendige Signalaufbereitung (Sequentisierung der Teilbilder) gegenüber den bisher bekannten Bauweisen aus. Das Endoskop in der grundsätzlichen Bauweise nach Anspruch 1 zeichnet sich durch seine schlanke Bauweise aus, insbesondere im distalen Bereich, da dort keine sperrigen Baukomponenten vorhanden sind. Der einzige Kamerakopf, ein CCD-Chip, ist ein räumlich kleines Bauteil (Anspruch 4). Das Anflanschen des Endoskops an eine geeignete leistungsfähige 3D-Elektronik wird in bewährter Technik nach der Lehre aus der DE 38 08 969 durchgeführt.

Sämtliche Komponenten des 3D-Endoskops in der einen oder anderen Bauweise sind vom Aufbau herkömmlicher 3D-Endoskope her bekannt und können bis auf den Einbau der beiden optischen Schalter, auch optische Ventile oder Shutter genannt, übernommen werden.

Im folgenden wird ein Ausführungsbeispiel des 3D-Video-Endoskops näher beschrieben. Es zeigen:
Figur 1 das 3D-Video-Endoskop mit elektrischem Übertragungskanal im Schaft,
Figur 2 ein V- oder Y-Prisma als Strahlumlenkungseinrichtung.

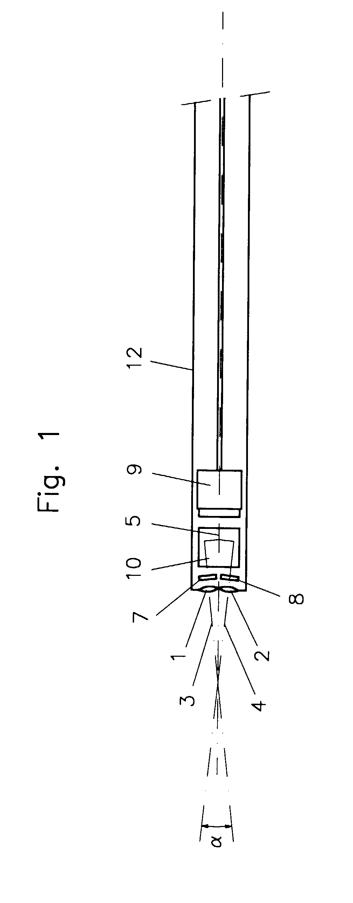

Die optisch zweikanalige Ausführung des 3D-Video-Endoskops gemäß Figur 1 ist für den gynäkologischen Einsatz konzipiert. Es besteht aus den beiden Vorlinsen 1, und 2, die das Objekt abbilden. Die optischen Achsen 3 und 4 der beiden im Abstand von etwa 2,7 mm nebeneinander liegenden Linsen 1 und 2 liegen in einer Ebene und schneiden einander unter dem vorgesehenen, für den stereoskopischen Eindruck maßgebenden Konvergenz- oder Schielwinkel a. Die Linsen 1, 2 haben in dieser Ausführung einen Durchmesser von 2,7 mm, so daß der optische Eingang des Endoskops einen größten äußeren Durchmesser von etwa 6,5 mm aufweist. Geringere Durchmesser werden angestrebt, um einen Einsatz als Operations- oder Diagnose-Endoskop für die Neurochirurgie oder Arthroskopie zu ermöglichen.

Im jeweiligen optischen Strahlengang schließen sich unmittelbar die beiden optischen Schalter 7, 8 an, die in der jeweiligen optischen Achse 3 bzw. 4 liegen. Im Durchführungsbeispiel sind diese Schalter 7 und 8 elektrooptische Blenden, die mit einer Frequenz von 100 bzw. 120 Hz komplementär abwechselnd transparent und opak gesteuert werden. Zur zuverlässigen und exakten Zuordnung werden sie über den Kamerakopf 9 angesteuert, über den die Bilder nacheinander ein- und ausgelesen werden.

Nach den beiden elektro-optischen Schaltern oder Blenden 7, 8 ist die Strahlumlenkeinrichtung 10 angeordnet, durch die hindurch stets nur ein Halbbild weitergeleitet und mit der einzigen Kamera 9 des Endoskops aufgenommen wird. Diese Kamera 9 ist eine CCD-Kamera (charge coupled device),d. h. mit ihr wird jedes an der Eintrittsfläche eintretende Bild in Bildelemente (pixel) zerlegt. Für die zuverlässige Zuordnung der Halbbilder wird zweckmäßigerweise die Steuerung der Blenden 7 und 8 direkt mit dem Takt der CCD-Kamera 9 gesteuert. Die weitere elektronische Verarbeitung der so gewonnenen Bildsignale ist z. B.in der DE 38 08 969 beschrieben.

Der Chip einer solchen CCD-Kamera von der Größe von 6 - 4 mm² (1/3"-Chip) hat 400 000 Bildpunkte brutto, von denen wenigstens 200 000 zur Bilddarstellung nach den heutigen Video-Standards ausgenutzt werden.

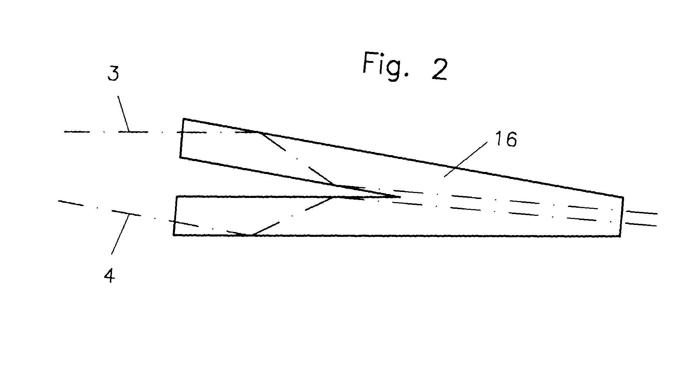

Beide Bauformen des 3D-Video-Endoskops mit den optischen Kanälen 6 nach Figur 1 und 2 liefern prinzipiell Bilder gleicher Qualität, da der Aufnahmevcrgang durch die Kamera und die Shuttersteuerung identisch ist. Im Schaft 12 können noch andere Einrichtungen wie Beleuchtung und/oder Saug- und Spülkanal untergebracht und mitgeführt werden, soweit die räumlichen Ver hältnisse das zulassen und der Schaftaufbau dadurch nicht verkompliziert wird und eine solche Kombination gefordert wird.

Die elektrischen Signale am Ausgang des Kamerakopfs 9 werden über eine elektrische Verbindung durch den Schaft 12 hindurch ausgelesen und auf einem Monitor zum Bild der Szene zusammengesetzt.

Ob der Schaft 12 steif oder flexibel ausgeführt ist, entscheidet sich am Einsatzfall. Der Vorteil der elektrischen Übertragung durch den Schaft 12 hindurch liegt in der unproblematischen elektrischen Signalübertragung. Der Schaft 12 ist mechanisch robust und kann daher sorgloser behandelt werden. Der Biegeradius des Schaftes kann sehr klein sein, ohne daß Bildpunkte ausfallen, wie das etwa bei Bildleitfaserbündel bei zu kleinen Biegeradien durch Bruch von einzelnen Fasern auftreten kann. Eine mit einem vorgegebenen Winkel a abgeknickte Schaftausführung ist somit einfach ohne besonderen konstruktiven Aufwand herstellbar.

Figur 2 zeigt den Aufbau der Strahlumlenkung 10 mit einem v- oder y-förmigen Prisma 16. Die beiden Schenkel des Prismas 16 sind an ihrer Mantelfläche verspiegelt und durch die Mitte des Fußes hindurch miteinander durch optischen Kitt verbunden. Beide Strahlenbündel werden beim Durchlauf durch ihren jeweiligen Schenkel an den beiden Schenkelwänden reflektiert und treten dann in der Fußfläche aus. Ein dabei durch die Reflektionsanordnung auftretender Versatz der beiden Strahlengänge ist zu berücksichtigen. Dieser Fehler fällt aber kaum ins Gewicht, weil er durch "Nachschielen" des Beobachters am Bildschirm kompensiert werden kann. Zum anderen ist durch Korrektur in der Horizontalphase in der Kameraelektronik dieser Versatz vollständig eliminierbar.

Eine vom herkömmlichen optischen Endoskopbau herrührende Bauvariante besteht darin, daß sich der rein optische Teil vom distalen Ende bis zum proximalen Bereich des Endoskops erstreckt, d. h. die Abbildung des Objekts erfolgt über die beiden Vorlinsen am distalen Ende, die über die beiden optischen Schalter auf den Eingang eines Bildleitfaserbündels abbilden. Am Ausgang des Bildleitfaserbündels, am Handhabungsbereich des Endoskops befindet sich die optoelektronische Aufnahmekamera, die die Halbbilder am Bildleitfaserausgang aufnimmt. Bei Verlegung der optischen Schalter in den Handhabungsbereich wären zwei getrennte Bildleitfaserbündel von der optischen Abbildung bis zur opto-elektronischen Kamera nötig.

### Bezugszeichenliste

- 1: Vorlinse
- 2: Vorlinse
- 3: optische Achse
- 4: optische Achse
- 5: optische Achse
- 6: optischer Kanal, Stablinsensystem, Bildleitfaserbündel
- 7: Schalter, Blende, Shutter
- 8: Schalter, Blende, Shutter
- 9: Kamerakopf, CCD-Kamera
- 10: Strahlumlenkeinrichtung
- 11: Prisma
- 12: Schaft, Endoskopschaft
- 13: 90°-Reflexionsprisma, Spiegel
- 14: Spiegelprisma
- 15: Kitt
- 16: Prisma

- α: Schielwinkel

## Patentansprüche

1. 3D-Video-Endoskop zum Betrachten von Gegenständen oder Oberflächen in Hohlräumen, bestehend aus:
a) zwei Vorlinsen (1, 2) mit je einer optischen Achse (3, 4);
b) je einem steuerbaren optischen Schalter (7, 8) im weiteren optischen Strahlengang, die beide abwechselnd im gleichen Rhythmus komplementär transparent bzw. opak gesteuert werden;
c) einer Strahlumlenkeinrichtung (10) im weiteren optischen Strahlengang, die die optischen Achsen (3, 4) der beiden Strahlengänge auf die optische Achse (5) einer optoelektronischen Aufnahmekamera (9) zusammenführt, wobei die Lichteintrittsfläche an der Aufnahmekamera (9) in der gemeinsamen Bildebene der beiden Vorlinsen (1, 2) liegt, und die Aufnahmekamera (9) im Rhythmus der beiden optischen Schalter (7, 8) einmal die elektrischen Signale des rechten und dann des linken Bildes zur weiteren Video-Bearbeitung bereitstellt,
dadurch gekennzeichnet, daß
i) sich die optischen Achsen (3, 4) der beiden Vorlinsen (1, 2) unter einem Winkel a schneiden,
ii) die optische Strahlumlenkeinrichtung (10) ein v- oder y-förmiges Strahlführungsgebilde ist, an dessen beide Schenkelstirnflächen je eine der beiden optischen Achsen (3, 4) eintritt, die optischen Achsen (3, 4) an den verspiegelten Wänden in dem Gebilde (10) je zweimal reflektiert werden und dann, zusammengeführt, am Fuße der Umlenkeinrichtung (10) austreten,
iii)sich die beiden, komplementär zueinander betriebenen optischen Schalter (7, 8) vor der Strahlumlenkeinrichtung (10) befinden.

2. 3D-Video-Endoskop nach Anspruch 1,
dadurch gekennzeichnet, daß
die Schaftachse (5) des Endoskops nach der opto-elektronischen Bildaufnahmeeinrichtung (9) auf der optischen Eingangsachse der Aufnahmekamera (9) oder in einem vorgegebenen Winkel dazu verläuft.

3. 3D-Video-Endoskop nach Anspruch 2,
dadurch gekennzeichnet, daß
die optischen Schalter (7, 8) elektrooptische Blenden sind.

4. 3D-Video-Endoskop nach Anspruch 3,
dadurch gekennzeichnet, daß
der Kamerakopf (9) ein CCD-Chip ist.

## Claims

1. 3D video endoscope for the observation of objects or surfaces in cavities, consisting of:
a) two preliminary lenses (1, 2) with an optical axis (3, 4) each;
b) a controllable optical switch (7, 8) each, which is located in the downstream optical beam path, and which are controlled alternately and complementarily at the same rhythm of transparent and opaque mode;
c) a beam deflection unit (10) located in the downstream optical beam path, which combines the optical axes (3, 4) of both beam paths on the optical axis (5) of an optoelectronic recording camera (9) with the light inlet area of the recording camera (9) lying on the common focal plane of the two preliminary lenses (1, 2) and the recording camera (9) supplying at the rhythm of both optical switches (7, 8) first the electric signals of the right and then of the left image for further video processing,
and characterized by
i) the optical axes (3, 4) of the two preliminary lenses (1, 2) intersecting each other under an angle a,
ii) the optical beam deflection unit (10) being a v- or y-shaped beam guiding system, where the optical axes (3, 4) enter the front surfaces of both legs, respectively, are reflected two times each on the mirror-coated walls of the system (10) and, after being combined, leave at the bottom of the deflection unit (10),
iii) both optical switches which are operated complementarily (7, 8) being located upstream of the beam deflection unit (10).

2. 3D video endoscope according to claim 1, characterized by the endoscope shaft axis (5) downstream of the optoelectronic image recording system (9) lying on the optical inlet axis of the recording camera (9) or under a given angle to it.

3. 3D video endoscope according to claim 2, characterized by the optical switches (7, 8) being electro-optical screens.

4. 3D video endoscope according to claim 3, characterized by the camera head (9) being a CCD chip.

## Revendications

1. Endoscope vidéo 3D pour l'analyse d'objets ou de surfaces dans des cavités, composé de :
a) deux lentilles avant (1, 2) composées chacune d'un axe optique (3, 4);
b) chacune d'elles étant munie d'un commutateur optique dirigeable (7, 8) dans la suite de la marche des rayons, tous les deux étant guidées alternativement au même rythme en mode complémentaire transparent ou respectivement opaque;
c) un dispositif de déviation de rayons (10) dans la suite de la marche optique des rayons, qui centre les axes optiques (3, 4) des deux marches de rayons sur l'axe optique d'une caméra de prise de vue optoélectronique (9), la surface d'entrée de lumière dans la caméra (9) se trouvant sur le plan focal commun des deux lentilles avant (1, 2), la caméra de prise de vue électronique (9) fournissant alternativement les signaux électriques de l'image droite et ensuite de l'image gauche, au rythme des deux commutateurs optiques (7, 8), en vue du traitement vidéo ultérieur,
caractérisé en ce que
i) les axes optiques (3, 4) des deux lentilles avant (1,2) se coupent sous un angle α,
ii) le dispositif de déviation des rayons optiques (10) est un ensemble de focalisation d'un faisceaux électronique en v ou y, à travers les deux faces de branches duquel vient entrer l'un des deux axes optiques respectifs (3, 4), où les axes optiques (3, 4) sont deux fois réfléchis chacun sur les deux faces réfléchissantes de l'ensemble (10) pour sortir ensuite, réunis, au pied du dispositif de déviation (10),
iii) les deux commutateurs optiques (7, 8), exploités en mode complémentaire, se trouvent en amont du dispositif de déviation des rayons (10).

2. Endoscope vidéo 3D selon la revendication 1,
caractérisé en ce que
l'axe de la tige (5) de l'endoscope en aval au dispositif de prise de vue opto-éléctronique (9) suit l'axe d'entrée optique de la caméra (9) ou un angle prédéfini par rapport à celui-ci.

3. Endoscope vidéo 3D selon la revendication 2,
caractérisé en ce que
les commutateurs optiques (7, 8) sont des diaphragmes électrooptiques.

4. Endoscope vidéo 3D selon la revendication 3,
caractérisé en ce que
la tête de la caméra (9) est un microprocesseur CCD.
